(19) Europäisches Patentamt
European Patent Office
Office européen des brevets

(11) Numéro de publication: 0 139 590
B1

# (12) FASCICULE DE BREVET EUROPEEN

(45) Date de publication du fascicule du brevet: **22.06.88**

(51) Int. Cl.[4]: **C 07 D 265/32**, A 61 K 31/535

(21) Numéro de dépôt: **84402065.1**

(22) Date de dépôt: **12.10.84**

(54) **4-Ethyl-2-hydroxy-3-méthyl-2-phénylmorpholine et ses sels d'addition.**

(30) Priorité: **17.10.83 FR 8316485**

(43) Date de publication de la demande: **02.05.85 Bulletin 85/18**

(45) Mention de la délivrance du brevet: **22.06.88 Bulletin 88/25**

(84) Etats contractants désignés: **AT BE CH DE FR GB IT LI LU NL SE**

(56) Documents cités:
**GB - A - 791 416**
**US - A - 3 117 967**
**US - A - 3 225 042**
**US - A - 4 044 131**

**JOURNAL OF HETEROCYCLIC CHEMISTRY, vol. 18, no. 3, mai 1981, pages 451-453, Hetero Corp.; D.R. MEYER et al.: "Heterocycles. 9. 2-Aryl-2H-1,4-tetrahydrooxazines (1,2)"**

(73) Titulaire: **LABORATOIRE L. LAFON Société anonyme dite:, 1 rue Georges Médéric, F-94701 Maisons-Alfort (FR)**

(72) Inventeur: **Lafon, Louis, 5 rue de l'Alboni, F-75016 Paris (FR)**

(74) Mandataire: **Clisci, Serge, S.A. FEDIT-LORIOT CONSEILS EN PROPRIETE INDUSTRIELLE 38, avenue Hoche, F-75008 Paris (FR)**

**EP 0 139 590 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne en tant que produits industriels nouveaux, des dérivés de 4-alkyl-2-hydroxy-3-méthyl-2-phényl-morpholine, à savoir la 4-éthyl-2-hydroxy-3-méthyl-2-phénylmorpholine et ses sels d'addition. Elle concerne également le procédé de préparation et l'utilisation en thérapeutique (notamment en tant qu'agents stimulants et anti-dépresseurs du SNC) de ces nouveaux composés.

On sait que l'on a déjà décrit un certain nombre de dérivés de 2-phényl-morpholine où le cycle morpholinyle est simultanément substitué en position 2 et en position 4. Parmi ces dérivés, on connaît des composés appartenant à la famille des 4-alkyl-2-phényl-morpholines notamment de GB-A-851 311, de US-A 2 997 469, de l'article de N. BUSCH et al., Eur. J. Med. Chem. Chimica Therapeutica 11 (No 3) pages 201–207 (1976), de FR-A 7 443M, de FR-B 2 111 882, de FR-A-2 471 378 et de EP-A-0 080 940. On sait également que plusieurs indications ont été envisagées ou préconisées pour les composés 2,4-disubstitués du type 4-alkyl-2-phényl-morpholine. Ces composés sont, notamment dans les publications précitées, présentés en tant qu'agents excitants ou stimulants du SNC, tranquillisants, sédatifs, anti-inflammatoires, analgésiques et hypotenseurs.

On connaît aussi de FR-A-1 535 615 des composés 2,2,4-trisubstitués, à savoir les 2,2-diméthyl-4-isoprophyl-morpholine, 2,2-diméthyl-4-[(3-méthyl)-butyl]-morpholine, 2,2-diméthyl-4-[(2-phényl)-éthyl]-morpholine et 2,2-diméthyl-4-[(2-phényl)-propyl]-morpholine.

On connaît enfin de US-A 3 117 967, des halogénures de 2-biphényl-2-hydroxy-morpholinium présentés en tant que substances antivirales susceptibles d'être substitués en position 3, 4 et 6 du squelette morpholinyle; de l'article de D.R. MEYER. Journal of Heterocyclic Chemistry 18 (No 3), pages 451–453, (1981), des composés 2,2,3,4-tétrasubstitués du type 3-alkyl-2-hydroxy-2-phényl-4-(2-hydroxyéthyl)-morpholine synthétisés en vue d'hypothétiques propriétés anoréxigènes; et de GB-A-791 416, la 3,4-diméthyl-2-hydroxy-2-phénylmorpholine en tant qu'intermédiaire de synthèse.

On vient de trouver de façon surprenante que les composés 2,2,3,4-tétrasubstitués selon l'invention, à savoir la 4-éthyl-2-hydroxy-3-méthyl-2-phénylmorpholine et ses sels d'addition, qui sont structurellement différents des composés antérieurement connus, sont encore plus intéressants que ces derniers en thérapeutique, notamment en tant qu'agents stimulants et antidépresseurs du SNC.

En particulier la 4-éthyl-2-hydroxy-3-méthyl-2-phénylmorpholine selon l'invention se distingue de la 3,4-diméthyl-2-hydroxy-2-phénylmorpholine sus-visée par (i) la présence sur l'atome d'azote du cycle morpholinyle d'un groupe éthyle au lieu d'un groupe méthyle, et (ii) l'absence d'effet tératogène.

Selon l'invention, on préconise donc en tant que produits industriels nouveaux, utiles en thérapeutique, la 4-éthyl-2-hydroxy-3-méthyl-2-phénylmorpholine de formule I

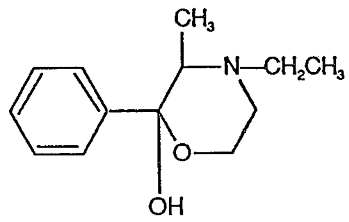

(I)

et ses sels d'addition (No de code = CRL 41 000).

Par sels d'addition, on entend ici, d'une part, les sels d'addition d'acide obtenus par réaction de la base libre de formule I avec des acides minéraux ou organiques, et, d'autre part, les sels d'ammonium. Parmi les acides utilisables pour salifier la base de formule I, on peut notamment citer les acides chlorhydrique, bromhydrique, acétique, formique, propionique, oxalique, fumarique, maléique, succinique, benzoïque, cinnamique, mandélique, citrique, malique, tartrique, aspartique, glutamique, méthanesulfonique, p-toluènesulfonique. Parmi les composés permettant d'obtenir des sels d'ammonium, on peut notamment citer $ICH_3$ et $ClCH_3$. D'une manière générale les sels d'addition d'acide sont préférés aux sels d'ammonium.

Le composé de formule I peut être préparé selon une méthode connue en soi par application de mécanismes réactionnels classiques. Le procédé que l'on préconise ici consiste à faire réagir l'α-bromopropiophénone de formule II

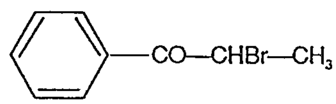

(II)

avec un excès, par rapport aux conditions stoechiométriques, de 2-éthylaminoéthanol de formule III

$CH_3CH_2-NH-CH_2-CH_2-OH$ (III)

dans un solvant inerte, notamment l'éther diméthylique ou diéthylique.

De façon avantageuse on utilisera environ 2 moles de III pour 1 mole de II, la durée de réaction étant comprise entre 4 et 24 heures et la température entre 5 et 25°C.

Selon l'invention on préconise une composition thérapeutique qui est caractérisée en ce qu'elle renferme, en association avec un excipient physiologiquement acceptable, le composé de formule I ou l'un de ses sels d'addition, en tant que principe actif.

Bien entendu, dans une telle composition le

principe actif, qui est choisi parmi l'ensemble constitué par le composé de formule I et ses sels non toxiques, intervient en quantité pharmaceutiquement efficace.

D'autres avantages et caractéristiques de l'invention seront mieux compris à la lecture qui va suivre d'un exemple de préparation et de résultats d'essais pharmacologiques, l'ensemble de ces éléments n'étant nullement limitatif mais donné à titre d'illustration.

Preparation

Obtention du chlorhydrate de 4-éthyl-2-hydroxy-3-méthyl-2-phényl-morpholine

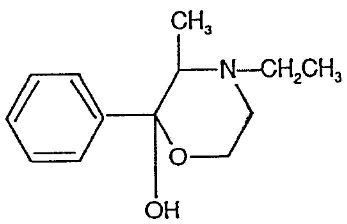

, HCl

(No de code: CRL 41 000)

Dans une solution de 41,78 g (0,469 mole) de 2-éthylaminoéthanol dans 800 ml d'éther anhydre, on coule goutte à goutte, 50 g (0,235 mole) d'α-bromopropiophénone. On laisse en contact 24 heures, coule 500 ml d'eau et décante la phase éthérée qu'on lave à l'eau. On extrait la phase éthérée par un mélange de 500 ml d'eau et 25 ml d'HCl concentré (d = 1,19), lave la phase aqueuse à l'éther. On alcalinise la phase aqueuse jusqu'à pH 11 au moyen de NaOH, extrait à l'éther, lave à l'eau et sèche la phase éthérée sur $MgSO_4$. On filtre, précipite le chlorhydrate au moyen d'éthanol chlorhydrique et recristallise les cristaux dans le mélange acétone-éthanol (1:1) v/v. On obtient 15 g (rendement: 25%) de CRL 41 000. F = 196°C (avec décomposition).

Analyse { % $Cl^{\ominus}$ mesuré : 14,02% ; % $Cl^{\ominus}$ théorique : 13,79%.

On a résumé ci-après les résultats des essais qui ont été entrepris avec le produit de l'exemple (CRL 41 000) qui est le composé préféré selon l'invention. Dans ces essais, en l'absence de précisions contraires, le CRL 41 000 a été administré en solution dans de l'eau distillée (pH 5), par voie intrapéritonéale, sous un volume de 20 ml/kg chez la souris mâle, et sous un volume de 5 ml/kg chez le rat mâle.

A-Toxicite

La DL-O (dose maximale non mortelle) chez la souris est supérieure à 256 mg/kg et inférieure à 512 mg/kg.

B-Etude neuropsychopharmacologique

I - Comportement global et reactivites

Des lots de 3 animaux sont observés avant, puis 15 min, 30 min, 1 h, 2 h, 3 h, et 24 h après l'administration de CRL 41 000.

1. Chez la souris, on observe aux doses suivantes:

128 mg/kg:
- une excitation avec présence de mouvements stéréotypés et hyperréactivité pendant 3 h, et
- une mydriase modérée pendant 2 h;

32 mg/kg:
- une excitation fugace (30 min).

2. Chez le rat, on observe aux doses suivantes:

64 mg/kg:
- une excitation avec augmentation de la réaction de peur et de la réactivité au toucher pendant 3 h,
- la présence de mouvements stéréotypés (3 h),
- une piloérection pendant 2 h, et
- une mydriase pendant 3 h;

16 mg/kg:
- des stéréotypies 2 h après administration, et
- une mydriase pendant 2 à 3 h;

4 mg/kg:
- une mydriase pendant 1 à 3 h.

II - Recherche de mouvements stereotypes

A la suite de l'observation de mouvements stéréotypés, parmi les symptômes notés, on a recherché les éventuelles stéréotypies que pourrait induire le CRL 41 000. Des groupes de 6 rats reçoivent le CRL 41 000 ou l'amphétamine immédiatement avant d'être placés dans des enceintes de petites dimensions où leur comportement stéréotypé est noté toutes les 10 min jusqu'à extinction de l'effet.

On constate que le CRL 41 000 induit chez le rat l'apparition de mouvements stéréotypés. L'intensité observée après 16 mg/kg de CRL 41 000 est comparable à celle obtenue après 2 mg/kg d'amphétamine.

III - Interaction avec l'apomorphine

1. Chez la souris

Des lots de 6 souris reçoivent le CRL 41 000 une demi-heure avant l'injection sous-cutanée de 1 ou 16 mg/kg d'apomorphine. On observe que le CRL 41 000, aux doses de 8 et surtout de 32 et de 128 mg/kg, s'oppose à l'action hypothermisante de l'apomorphine, sans modifier le comportement de verticalisation et les stéréotypies.

2. Chez le rat

Le CRL 41 000 est administré à des lots de 6 rats une demi-heure avant l'injection sous-cutanée de 0,5 mg/kg d'apomorphine. On constate que, à forte dose (64 mg/kg), le CRL 41 000 entraîne une potentialisation des stéréotypies induites par l'apomorphine.

IV - Interaction avec l'amphetamine

L'amphétamine (2 mg/kg) est injectée par voie intrapéritonéale à des lots de 6 rats, une demi-heure après l'administration de CRL 41 000. On note que, aux doses de 16 et 64 mg/kg, le CRL

41 000 potentialise nettement les stéréotypies amphétaminiques.

V - Interaction avec la reserpine

Quatre heures après l'injection intrapéritonéale de 2,5 mg/kg de réserpine, des lots de 6 souris reçoivent le CRL 41 000. On observe l'action du produit à tester vis-à-vis de l'hypothermie et du ptôsis induits par la réserpine. On constate que, aux doses de 8 mg/kg, 32 mg/kg et 128 mg/kg, le CRL 41 000 s'oppose à l'hypothermie réserpinique. L'intensité du ptôsis réserpinique est diminuée aux doses de 32 mg/kg et 128 mg/kg et CRL 41 000.

VI - Interaction avec l'oxotremorine

Le CRL 41 000 est administré à des lots de 6 souris une demi-heure avant l'injection intrapéritonéale de 0,5 mg/kg d'oxotrémorine.

1. Action sur la température

Aux doses de 2 mg/kg et 8 mg/kg mais surtout de 32 mg/kg et de 128 mg/kg le CRL 41 000 s'oppose à l'action hypothermisante de l'oxotrémorine.

2. Action sur les tremblements

Aux doses de 32 mg/kg et 128 mg/kg le CRL 41 000 diminue l'intensité des tremblements dûs à l'oxotrémorine.

3. Action sur les symptômes cholinergiques périphériques

Le CRL 41 000 ne modifie pas de façon sensible les signes de stimulation cholinergique périphérique provoqués par l'oxotrémorine.

VII - Action sur le test des quatre plaques, la traction et l'electrochoc

Le test est pratiqué sur des lots de 10 souris, une demi-heure après l'administration de CRL 41 000. On observe que, aux doses de 8 mg/kg, 32 mg/kg et 128 mg/kg, le CRL 41 000 entraîne une augmentation du nombre de passages punis, qu'il ne provoque pas d'incapacité motrice majeure et que, à forte dose (128 mg/kg), il s'oppose aux effets convulsivants de l'électrochoc.

VIII - Action sur la motilite spontanee

Une demi-heure après avoir reçu le CRL 41 000, les souris sont placées en actimètre où leur motilité est enregistrée pendant 30 min. On constate que, aux doses les plus fortes qui ont été utilisées (64 mg/kg et 128 mg/kg), le CRL 41 000 provoque une augmentation très nette de l'activité motrice spontanée de la souris.

IX - Action sur l'agressivite intergroupes

Après avoir séjourné pendant 3 semaines dans chacune des moitiés d'une cage séparée par une cloison opaque, des groupes de 3 souris reçoivent le CRL 41 000. Une demi-heure plus tard, les deux groupes d'une même cage sont réunis par retrait de la cloison et on note le nombre de combats qui surviennent en 10 min. On constate que, à forte dose (128 mg/kg), le CRL 41 000 entraîne une diminution nette du nombre de combats.

X - Action vis-à-vis de quelques comportements perturbes par divers agents

1. Motilité réduite par habituation à l'enceinte

Après 18 h de séjour dans les actimètres, les souris (6 par dose, 12 témoins) reçoivent le CRL 41 000. Elles sont aussitôt replacées dans leurs enceintes respectives et, une demi-heure plus tard, on enregistre leur motilité pendant 30 min.

On constate que, aux doses de 32 mg/kg et surtout 128 mg/kg, le CRL 41 000 provoque une reprise de l'activité motrice chez la souris habituée à son enceinte.

2. Motilité réduite par agression hypoxique

Une demi-heure après avoir reçu le CRL 41 000, les souris (10 par dose, 20 témoins) sont soumises à une anoxie hypobara aiguë [dépression de 600 mmHg (soit environ $8 \times 10^4$ pascals) en 90 s; détente de 45 s], puis elles sont placées en actimètre où leur motilité est enregistrée pendant 10 min.

On observe que, à forte dose (128 mg/kg), le CRL 41 000 entraîne une amélioration très sensible de la récupération motrice chez les souris dont la motilité a été déprimée à la suite d'un séjour bref dans une enceinte à pression réduite.

3. Anoxie asphyxique

Des lots de 10 souris reçoivent le CRL 41 000 une demi-heure avant l'administration intrapéritonéale de 32 mg/kg de triiodoéthylate de gallamine (agents curarisant de référence). On constate que le CRL 41 000 n'entraîne pas de modifications du délai d'apparition des convulsions et de la mort consécutives à une anoxie asphyxique provoquée par un curarisant.

XI - Interaction avec le barbital

Une demi-heure après l'administration de CRL 41 000, des lots de 10 souris reçoivent une injection intrapéritonéale de barbital (220 mg/kg).

Dès la dose de 2 mg/kg le CRL 41 000 diminue la durée du sommeil barbiturique. L'effet est maximal aux doses de 32 à 128 mg/kg.

XII - Action sur le «desespoir comportemental»

Une demi-heure après avoir reçu le CRL 41 000, des lots de 6 souris sont placés dans un bécher rempli d'eau sur une hauteur de 6 cm. On note la durée totale d'immobilité entre la 2e et la 6e minutes suivant l'immension. On observe que, aux doses de 32 mg/kg et de 128 mg/kg, le CRL 41 000 entraîne une diminution nette de la durée de l'immobilité dite de «désespoir».

XIII - Recherche d'une toxicite particuliere chez les souris groupées

Aussitôt après l'administration de CRL 41 000 les souris groupées par lots de 10, sont placées dans des cages en Plexiglass ($20 \times 10 \times 10$ cm). On note le nombre d'animaux morts, toutes les heures pendant 4 heures et après 24 heures. La toxicité du CRL 41 000 est déterminée dans les mêmes conditions sur des lots de 10 souris placées à raison d'une par cage.

Dans ces conditions on observe une absence de toxicité particulière chez les souris groupées,

le rapport R = DL-50 souris isolées/(DL-50 souris groupées étant inférieur ou égal à 2. Pour comparaison, dans les mêmes conditions le rapport R est de 8 pour l'amphétamine et le diéthylpropion, de 6 pour le méthylyphénidate, de 4 pour la benzphétamine et de 3 pour la nomifensine.

XIV – Activité antidepressive par voie orale

On a étudié, selon deux tests prévisionnels (intéraction avec l'apomorphine et intéraction avec la réserpine) les effets antidépresseurs du CRL 41 000 administré par voie orale chez la souris mâle (le CRL 41 000 étant dans ces deux tests administré en solution dans de l'eau distillée au moyen d'une sonde gastrique sous un volume de 20 mg/kg).

Dans ces tests le CRL 41 000 s'est révélé très actif en tant qu'agent antidépresseur.

Par ailleurs au cours des essais cliniques entrepris chez l'homme l'activité antidépressive du CRL 41 000 a été obtenue par voie orale.

C. Etude cardiovasculaire

XV – Action chez le rat démédullé

Le CRL 41 000 administré en solution aqueuse dans de l'eau distillée, par voie intraveineuse, a été comparé à l'amphétamine et à la tyramine selon trois technique différentes chez le rat démédullé. Les résultats obtenus [variation de la pression artérielle systolique ΔPA en mm Hg (1 mm Hg correspond approximativement à $1{,}333 \times 10^2$ pascals)] ont été consignés dans les Tableaux I, II et III ci-après.

Les résultats des Tableaux I, II et III montrent que

a) l'activité hypertensive de la tyramine est totalement bloquée après pré-traitement avec la réserpine, alors qu'elle n'est pas modifiée par pré-traitement avec l'α-méthyltyrosine;

b) l'activité hypertensive de l'amphétamine n'est pas totalement bloquée par la tyramine (déplétion en catécholamines), par l'α-méthyltyrosine (blocage de la synthèse des catécholamines) et par l'association de ces deux réactifs;

c) en revanche l'activité hypertensive (2è phase progressive et de durée supérieure ou égale à 1 h) du CRL 41 000 est totalement bloqueé par la tyramine, l'α-méthyltyrosine, et l'association des deux réacitifs.

Variation de la pression arterielle systolique (Δ PA) chez le rat demedulle*

Tableau I

Après déplétion en catécholamines par pré-traitement au moyen de réserpine (7,5 mg/kg I.P. 20 h avant administration des produits à tester)

| | Tyramine 200 µg/kg I.V. | Amphétamine 250 µg/kg I.V. | CRL 41000 30 mg/kg I.V. | |
|---|---|---|---|---|
| | | | 1ère phase | 2è phase |
| Δ PA (mm Hg) | 0 | + 49 | + 30 | 0 |

Tableau II

Après blocage de la synthèse des catécholamines par pré-traitement au moyen d'α-méthyltyrosine (250 mg/kg I.P. 20 h avant, puis 250 mg/kg I.P. 2 h avant administration des produits à tester)

| | Tyramine 200 µg/kg I.V. | Amphétamine 250 µg/kg I.V. | CRL 41000 30 mg/kg I.V. | |
|---|---|---|---|---|
| | | | 1ère phase | 2è phase |
| Δ PA (mm Hg) | + 19 | + 17 | + 24 | 0 |

Tableau III

Après blocage de la synthèse par α-méthyltyrosine et déplétion par réserpine administrées, selon les modalités données ci-dessus, avant les produits à tester

| | Tyramine 200 µg/kg I.V. | Amphétamine 250 µg/kg I.V. | CRL 41000 30 mg/kg I.V. | |
|---|---|---|---|---|
| | | | 1ère phase | 2è phase |
| Δ PA (mm Hg) | 0 | + 20 | + 60 | 0 |

Note: * sur lots de 3 animaux par produit à tester.

D. Etude teratogenique

Les essais entrepris chez le lapin (lot de 10 animaux femelles par dose; 15 témoins) selon un protocole comprenant

- administration par gavage gastrique de CRL 41 000 aux doses quotidiennes de 0, 50, 150 et 200 mg/kg du 5è au 18è jour de la gestation, puis
- césarienne effectuée le 29è jour de la gestation, ont mis en évidence que le CRL 41 000, à la différence de certains dérivés de 2-phényl-morpholine antérieurement connus, est dépourvu d'effet tératogène.

E - Conclusions

Il résulte des résultats donnés ci-dessus que le CRL 41 000 présente un profil d'agent stimulant du SNC (excitation chez la souris et le rat, hyperréactivité, augmentation du nombre de passages punis selon le test des quatre plaques, reprise de l'activité motrice chez la souris habituée à son enceinte, présences de mouvements stéréotypés - notamment potentialisation des stéréotypies induites par l'apomorphine et l'amphétamine –, antagonisme du sommeil barbiturique).

A côté de ces effets stimulants, le CRL 41 000 présente des effets anti-dépresseurs (antagonisme des hypothermies induites par l'apomorphine, la réserpine et l'oxotrémorine, d'une part, et diminution de la durée d'immobilité dite de «désespoir»).

Ces propriétés stimulantes et anti-dépressives suggèrent que le CRL 41 000 agit comme les substances amphétaminiques, avec en particulier deux différences d'effet (toxicité groupée et activité hpyertensive).

En clinique, on a observé que le CRL 41 000 a donné, en tant qu'agent anti-dépresseur, de bons résultats chez l'homme quand il a été administré par voie orale sous forme de comprimés ou de gélules (renfermant chacun 10 mg dudit CRL 41 000) à raison de 2 à 3 comprimés ou gélules par jour, dans le traitement des états dépressifs.

**Revendications pour les états contractants BE, CH, DE, FR, GB, IT, LI, LU, NL, SE**

1. 4-Ethyl-2-hydroxy-3-méthyl-2-phénylmorpholine de formule I

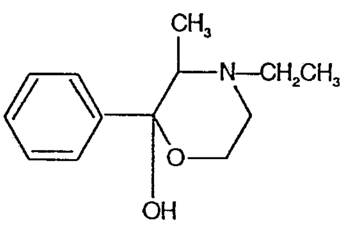 (I)

et ses sels d'addition.

2. Chlorhydrate de 4-éthyl-2-hydroxy-3-méthyl-2-phénylmorpholine.

3. Composition thérapeutique, caractérisée en ce qu'elle renferme, en association avec un excipient physiologiquement acceptable au moins un dérivé choisi parmi la 4-éthyl-2-hydroxy-3-méthyl-2-phénylmorpholine et ses sels d'addition non toxiques.

4. Procédé de préparation de la 4-éthyl-2-hydroxy-3-méthyl-2-phénylmorpholine, caractérisé en ce que l'on fait réagir l'α-bromopropiophénone de formule II

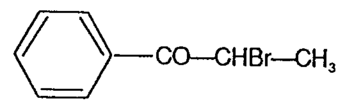 (II)

avec le 2-éthylaminoéthanol de formule II

$CH_3CH_2-NH-CH_2-CH_2-OH$ (III)

à raison de 2 moles environ de III pour 1 mole de II, à une température comprise entre 5 et 25°C, pendant 4 à 24 heures.

**Revendications pour l'état contractant AT**

1. Procédé de préparation de la 4-éthyl-2-hydroxy-3-méthyl-2-phénylmorpholine de formule I

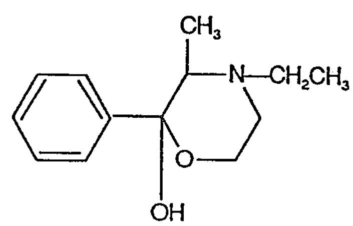 (I)

et de ses sels d'addition, caractérisé en ce que l' on fait réagir l'α-bromopropiophénone de formule II

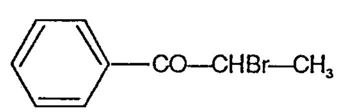 (II)

avec un excès, par rapport aux conditions stoechiométriques, de 2-éthylaminoéthanol de formule III

$CH_3CH_2-NH-CH_2-CH_2-OH$ III

2. Procédé suivant la revendication 1, caractérisé en ce que l'on fait réagir environ 2 moles de 2-éthylaminoéthanol avec environ 1 mole d'α-bromopropiophénone, à une température comprise entre 5 et 25°C, pendant 4 à 24 heures.

**Patentansprüche für die Vertragsstaaten BE, CH, DE, FR, GB, IT, LI, LU, NL, SE**

1. 4-Ethyl-2-hydroxy-3-methyl-2-phenylmorpholin der Formel I

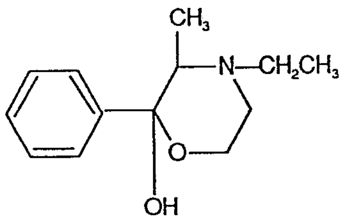

(I)

und seine Additionssalze.

2. Chlorhydrat des 4-Ethyl-2-hydroxy-3-methyl-2-phenyl-morpholins.

3. Therapeutische Zusammensetzung, dadurch gekennzeichnet, dass sie in Verbindung mit einem physiologisch akzeptablen Träger mindestens eine Verbindung aus der Gruppe 4-Ethyl-2-hydroxy-3-methyl-2-phenylmorpholin und seiner nicht toxischen Additionssalze enthält.

4. Verfahren zur Herstellung von 4-Ethyl-2-hydroxy-3-methyl-2-phenylmorpholin, dadurch gekennzeichnet, dass man l'α-Bromopropiophenon der Formel II

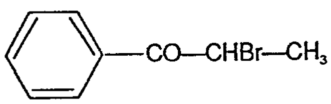

(II)

mit 2-Ethylaminoethanol der Formel III

$CH_3CH_2$-NH-$CH_2$-$CH_2$-OH III

im Verhältnis von ungefähr 2 Mol von III zu 1 Mol von II, bei einer Temperatur zwischen 5 und 25°C, während 4 bis 24 Stunden reagieren lässt.

**Patentansprüche für den Vertragsstaat AT**

1. Verfahren zur Herstellung von 4-Ethyl-2-hydroxy-3-methyl-2-phenylmorpholine der Formel I

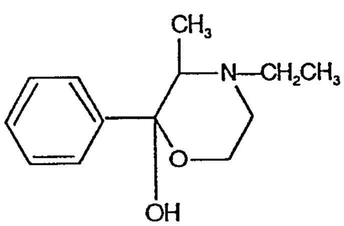

(I)

und seiner Additionssalze, dadurch gekennzeichnet, dass man l'α-Bromopropiophenon der Formel II

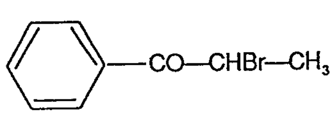

(II)

mit einem Überschuss, bezogen auf die stöchiometrischen Bedingungen, an 2-Ethylaminoethanol der Formel III

$CH_3CH_2$-NH-$CH_2$-$CH_2$-OH (III)

reagieren lässt.

2. Verfahren nach Anspruch 1, dadurch gekennzeichnet, dass man ungefähr 2 Mol 2-Ethylaminoethanol mit ungefähr 1 Mol l'α-Bromopropiophenon bei einer Temperatur zwischen 5 und 25°C während 4 bis 24 Stunden reagieren lässt.

**Claims for the contracting states BE, CH, DE, FR, GB, IT, LI, LU, NL, SE**

1. 4-Ethyl-2-hydroxy-3-methyl-2-phenylmorpholine of formula I

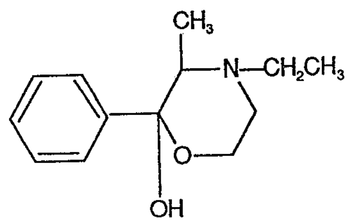

(I)

and its addition salts.

2. 4-Ethyl-2-hydroxy-3-methyl-2-phenylmorpholine hydrochloride.

3. Therapeutic composition, characterized in that it contains, in combination with a physiologically acceptable excipient, at least one derivative chosen from 4-ethyl-2-hydroxy-3-methyl-2-phenylmorpholine and its non-toxic addition salts.

4. Process for preparing 4-ethyl-2-hydroxy-3-methyl-2-phenylmorpholine, characterized in that α-bromopropiophenone of formule II

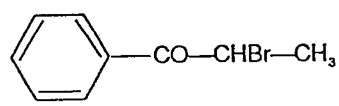

(II)

is reacted with 2-ethylaminoethanol of formula III

$CH_3CH_2$-NH-$CH_2$-$CH_2$-OH (III)

in the proportion of approximately 2 moles of III for 1 mole of II, at a temperature of between 5 and 25°C, for 4 for 24 hours.

**Claims for the contracting state AT**

1. Process for preparing 4-ethyl-2-hydroxy-3-methyl-2-phenylmorpholine of formula I

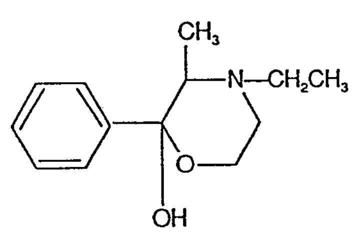

and its addition salts, characterized in that α-bromopropiophenone of formula II

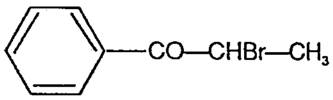

is reacted with an excess, with respect to the stoichiometric conditions, of 2-ethylaminoethanol of formula III

$CH_3CH_2-NH-CH_2-CH^2-OH$ (III)

2. Process according to Claim 1, characterized in that approximately 2 moles of 2-ethylaminoethanol are reacted with approximately 1 mole of α-bromopropiophenone, at a temperature of between 5 and 25°C, for 4 to 24 hours.